# EUROPEAN PATENT SPECIFICATION

(11) **EP 2 669 384 B1**
(45) Date of publication and mention of the grant of the patent: **09.08.2017**
(21) Application number: 12739047.4
(22) Date of filing: 24.01.2012
(51) Int. Cl.: C12Q 1/68, C12Q 1/02, G01N 33/50, G01N 33/68

(54) **METHOD FOR EVALUATING THE PRESENCE OF RHEUMATOID ARTHRITIS AND BIOMARKER SET USED IN THE SAME**
VERFAHREN ZUR BEWERTUNG DER ANWESENHEIT VON RHEUMATOIDER ARTHRITIS UND IN DIESEM VERFAHREN VERWENDETER BIOMARKERSATZ
MÉTHODE POUR ÉVALUER LA PRÉSENCE DE LA POLYARTHRITE RHUMATOÏDE ET ENSEMBLE DE BIOMARQUEURS UTILISÉ POUR CE FAIRE

(30) Priority: 28.01.2011 JP 2011016739
(43) Date of publication of application: 04.12.2013
(73) Proprietor: Sysmex Corporation, Kobe-shi, Hyogo 651-0073 (JP)
(72) Inventor: UGA, Hitoshi, Kobe-shi Hyogo 651-0073 (JP); MIYAMOTO, Yoshiaki, Kobe-shi Hyogo 651-0073 (JP); IKEDA, Masafumi, Kobe-shi Hyogo 651-0073 (JP); KURATA, Hirokazu, Kobe-shi Hyogo 651-0073 (JP)
(74) Representative: Paemen, Liesbet R.J.
(86) International application number: PCT/JP2012/000423
(87) International publication number: WO 2012/102020

(56) References cited:
- WO-A1-2011/013753
- DE-A1- 10 328 033
- JP-A- 2006 503 587
- US-A1- 2003 228 617
- US-A1- 2007 292 881
- US-A1- 2010 273 671
- "AFFYMETRIX GENECHIP HUMAN GENOME U.133", GEO - GENE EXPRESSION OMNIBUS, , 11 March 2002 (2002-03-11), XP008136197, Retrieved from the Internet: URL:http://www.ncbi.nlm.nih.gov/geo/query/ acc.cgi?acc=GPL96
- LI Q ET AL: "Characterization of the transdermal transport of flurbiprofen and indomethacin", JOURNAL OF CONTROLLED RELEASE, ELSEVIER, AMSTERDAM, NL, vol. 110, no. 3, 21 February 2006 (2006-02-21), pages 542-556, XP024957411, ISSN: 0168-3659, DOI: 10.1016/J.JCONREL.2005.09.054 [retrieved on 2006-02-21]
- DATABASE CAPLUS [Online] 'GENE EXPRESSION PROFILE OF PERIPHERAL BLOOD CD4 POSITIVE T CELLS IN RHEUMATOID ARTHRITIS', XP003030282 Retrieved from STN Database accession no. 2007:1250623 & ZHONGHUA FENGSHIBINGXUE ZAZHI vol. 10, no. 7, 2006, pages 438 - 439
- LI J. ET AL.: 'Altered microRNA expression profile with miR-146a upregulation in CD4+ T cells from patients with rheumatoid arthritis' ARTHRITIS RES THER., [Online] vol. 12, no. 3:R81, 11 May 2010, pages 1 - 12, XP021085209 Retrieved from the Internet: <URL:http://www.ncbi.nlm.nih.gov/pmc/articl es/PMC2911863/pdf/ar3006.pdf> [retrieved on 2012-04-16]

## Description

### TECHNICAL FIELD

The present invention relates to a method for evaluating presence of rheumatoid arthritis, and a biomarker set used in the method.

### BACKGROUND ART

Chronic rheumatoid arthritis (rheumatoid arthritis: hereinafter, referred to as "RA") is a systemic inflammatory autoimmune disease whose main clinical symptom is arthritis. In RA, in addition to joint pains, destructions of cartilages and bones also occur. These destructions are associated with a deformed joint structure, resulting in restricted range of joint motion. This significantly deteriorates the RA patient's Quality of Life. In addition, it has been reported that life prognosis of a RA patient is approximately 10 years shorter when compared to that of a healthy individual.

It is known that joint destruction in RA rapidly progresses within 2 years of onset. On the other hand, this stage is referred to as a stage most requiring treatment in order to induce remission (Window of Opportunity), since therapeutic sensitivity to an antirheumatic agent is high in this stage. Therefore, for medical care of RA, it is important to accurately provide a diagnosis at an early stage and immediately execute an aggressive treatment.

Conventionally, RA is diagnosed through subjective symptoms of joint pains and the like, and physical observations based on the degree of joint swelling, bone X-ray observations, and the like. However, methods using such physical observations lead to variability in the result of diagnosis, depending on the experience and skill of a physician who is performing the medical examination. Furthermore, there has been a problem of requiring a long period of time to obtain the diagnosis.

Further objective methods include a method based on a serological test of autoantibodies such as rheumatoid factor (hereinafter, referred to as "RF") and anti-cyclic citrullinated peptide antibody (hereinafter, referred to as "anti-CCP antibody"). These test observations are also used in the ACR/EULAR diagnostic criteria proposed by the American College of Rheumatology (ACR) and the European League Against Rheumatism (EULAR).

RF is an autoantibody against IgG, and approximately 80% of RA patients test positive for that. However, since approximately 20% of RA patients test negative for RF whereas some healthy individuals also test positive, it is not possible to distinguish between healthy individuals and RA patients with high accuracy by RF alone.

Anti-CCP antibody is an autoantibody that can be measured using a cyclic citrullinated peptide as an antigen, and is considered to have higher sensitivity and specificity against RA than those of RF. Therefore, anti-CCP antibody has gathered attention as a factor for early diagnosis and prognosis prediction of RA, and is used in the country and overseas for the diagnosis of RA.

However, it has been reported that, even when tests are conducted using anti-CCP antibody which is considered clinically useful, variability has been observed in sensitivity depending on the testing method and the type of antigen that has been used (Non-Patent Literature 1). There have been many cases where the sensitivities were 40 to 60%, and the test with anti-CCP antibody cannot be considered to be sufficiently accurate.

Thus, methods for detecting autoimmune diseases including RA based on expression of biomarkers other than autoantibodies have been proposed. For example, Patent Literature 1 discloses, as biomarkers for detecting autoimmune diseases, 35 genes including SLC16A4 (solute carrier family 16, member 4) which is a gene whose expression is lowest in systemic lupus erythematosus (SLE) patients. Patent Literature 2 discloses approximately 70 genes including SLCO2B1 (solute carrier organic anion transporter family, member 2B1), as genes highly expressed in association with rheumatoid arthritis. Patent Literature 3 discloses 2059 genes including PTPRM (protein tyrosine phosphatase, receptor type, M) as genes exhibiting expressional difference in arthritis diseases such as SLE, seronegative arthritis (SA), osteoarthritis (OA), and RA.

### CITATION LIST

### [PATENT LITERATURE]

[PTL 1] Japanese Unexamined Patent Publication (Translation of PCT Application) No. 2006-503587, corresponding to WO2004/046098.
[PTL 2] US 2007/0292881
[PTL 3] US 2010/0273671

### [NON PATENT LITERATURE]

[NPL 1] Tsuneyo Mimori, "Other autoantibodies in rheumatoid arthritis (anti-citrullinated proteins and anti-calpastatin antibodies)," Nippon Rinsho, Vol.66, Suppl 5, 2010

### SUMMARY OF THE INVENTION

### PROBLEMS TO BE SOLVED BY THE INVENTION

As described above, for the medical care of RA, it is demanded to accurately diagnose RA in order not to miss the window of therapeutic opportunity (window of opportunity). For this, further development is desired for a new biomarker set that has higher accuracy and is useful for providing diagnosis of RA, and an evaluation method of RA using such a marker set.

An object of the present invention is to seek a new biomarker set capable of distinguishing between RA patients and healthy individuals with high accuracy, and provide a method for evaluating presence of rheumatoid arthritis using the new marker set.

### SOLUTION TO THE PROBLEMS

The present inventors have focused on IL-17 producing helper T (Th17) cells that are considered to be a cause of autoimmune diseases including RA, and identified genes that are specifically expressed in Th17 cells isolated from peripheral blood of healthy adults (International application WO20111013753). For the present invention, among those genes, the present inventor have extracted 9 genes that are highly expressed in RA patients, and discovered a combination of the genes enabling distinguishing between healthy individuals and RA patients with high accuracy to accomplish the present invention.

Therefore, the present invention provides a method for evaluating presence of rheumatoid arthritis, the method comprising the steps of:
acquiring, from a biological specimen obtained from a subject, information regarding expression level of a first biomarker which is SLC16A4, and information regarding expression level of at least one second biomarker which is SLCO2B1 ; and
evaluating presence of rheumatoid arthritis in the subject based on the information regarding expression levels of the acquired first and second biomarkers.

In addition, the present disclosure provides a biomarker set for evaluating presence of rheumatoid arthritis in a live body, including the first biomarker and the second biomarker.

### ADVANTAGEOUS EFFECTS OF THE INVENTION

With the method for evaluating presence of rheumatoid arthritis of the present invention, presence of rheumatoid arthritis can be evaluated in subjects with high accuracy.

### BRIEF DESCRIPTION OF THE DRAWINGS

[FIG. 1] FIG. 1 is a scatter diagram showing expression levels in healthy individual group and RA patient group, regarding 9 genes identified as genes that vary significantly between the healthy individual group and the RA patient group.
[FIG. 2] FIG. 2 is a scatter diagram showing predictive values of the healthy individual group and the RA patient group for a biomarker set of the present invention.
[FIG. 3] FIG. 3 is a scatter diagram showing a predictive value of the healthy individual group and anti-CCP antibody negative RA patient group for a biomarker set of the present invention.

### DESCRIPTION OF EMBODIMENTS

### [1. Method for Evaluating Presence of Rheumatoid Arthritis]

In a method for evaluating presence of RA (hereinafter, also simply referred to as "evaluation method") of the present invention, first, from a biological specimen obtained from a subject, information regarding expression level of a first biomarker which is SLC16A4, and information regarding expression level of at least one second biomarker which is SLCO2B1 are acquired.

In embodiments of the present invention, the subject is not particularly limited, and examples thereof include healthy individuals, individuals suspected to be affected with RA, and RA patients.

In embodiments of the present invention, the biological specimen is not particularly limited as long as it is a specimen collected from a subject, containing a biological material. Such a biological material includes cells, nucleic acids, proteins, and the like. The biological specimen is preferably a specimen containing cells. Specific examples of the biological specimen include blood, synovial fluid, cerebrospinal fluid, pleural fluid, ascites, lymph fluid, and tissues and cells collected through surgery or biopsy. Among those, a specimen containing cells in the blood is preferable.

As described later, in embodiments of the present invention, the first and second biomarkers may be polynucleotide markers or polypeptide markers. Therefore, when acquiring information regarding expression levels of the first and second biomarkers that are polynucleotide markers, nucleic acid is extracted from the biological specimen. Extraction of nucleic acid from the biological specimen can be conducted in accordance with a method known in the art. The extraction can be conducted by, for example, mixing the biological specimen with a process liquid, which contains a surfactant (e.g., sodium cholate, sodium dodecyl sulfate, etc.), for solubilizing cells and tissues therein, and applying a physical treatment (stirring, homogenization, ultrasonic wave fragmentation, etc.) on the obtained mixture to allow release of, in the mixture, nucleic acid contained in the biological specimen. In this case, the mixture is centrifuged to precipitate cell debris for collecting a supernatant containing nucleic acid, and this supernatant is preferably used as the biological specimen. In addition, a method known in the art may be used to purify nucleic acid from the obtained supernatant. The extraction and purification of nucleic acid from the biological specimen may be conducted using a commercially available kit.

When acquiring information regarding expression levels of the first and second biomarkers that are polypeptide markers, protein is extracted from the biological specimen. Extraction of protein from the biological specimen can be conducted in accordance with a method known in the art. The extraction can be conducted by, for example, homogenizing cells contained in the biological specimen with ultrasonic waves or mixing the biological specimen with a cell solubilization liquid, and applying a physical treatment (stirring, homogenization, ultrasonic wave fragmentation, etc.) on the obtained mixture to allow release of, in the mixture, protein contained in the biological specimen. In this case, preferably, the mixture is centrifuged to precipitate cell debris for collecting a supernatant containing protein, and this supernatant is preferably used as the biological specimen.

The base sequences and amino acid sequences of the first and second biomarkers can be obtained from databases such as UniGene provided by the National Center for Biotechnology Information (NCBI). Accession numbers of the respective biomarkers are shown in the following Table 1. It should be noted that, these accession numbers are the latest numbers at the date of January 19, 2011.

**[Table 1]**

| Gene Symbol | Entrez Gene ID | Protein ID | Transcript ID | UniGene ID |
|---|---|---|---|---|
| SLC16A4 | 9122 | NP_004687 | NM_004696 | Hs.351306 |
| SLCO2B1 | 11309 | NP_009187 | NM_007256 | Hs.7884 |
| PTPRM | 5797 | NP_001098714, NP_002836 | NM_001105244, M_002845 | Hs.49774 |
| SHB | 6461 | NP_003019 | NM_003028 | Hs.521482 |
| ATP9A | 10079 | NP_006036 | NM_006045 | Hs.726016 |

In embodiments of the present invention, the first and second biomarkers may be polynucleotide markers or polypeptide markers.

Therefore, the first biomarker is a polynucleotide having a base sequence of a gene designated as SLC16A4, a mutated form thereof, or a fragment of those, or the first biomarker is a protein encoded by a gene designated as SLC16A4, a functionally equivalent mutated form thereof, or a fragment of those.

Furthermore, the second biomarker is a polynucleotide having a base sequence of a gene designated as at least SLCO2B1, a mutated form thereof, or a fragment of those; or the second biomarker is a protein encoded by a gene designated as at least SLCO2B1, a functionally equivalent mutated form thereof, or a fragment of those.

It should be noted that, in the present specification, the polynucleotide may be either DNA or RNA, or may be the above described gene (DNA) itself, or mRNA, cDNA, or cRNA thereof.

As used herein, a mutated form of a polynucleotide refers to a polynucleotide having introduced therein a mutation while not altering the function of a protein encoded by the gene. Such mutation includes deletion or substitution of one or more nucleotides, or addition of one or more nucleotides to a base sequence of the gene. The sequence identity of a base sequence of the mutated form with respect to a base sequence of the original gene is at least 80% or higher, preferably at least 85% or higher, more preferably at least 90% or higher, and particularly preferably at least 95% or higher.

As used herein, a functionally equivalent mutated form of a protein refers to a protein having introduced therein a mutation while not altering the function of the protein. Such mutation includes deletion or substitution of one or more amino acid residues, or addition of one or more amino acid residues to an amino acid sequence of the protein. The sequence identity of an amino acid sequence of the mutated form that is functionally equivalent to the protein with respect to an amino acid sequence of the original protein is at least 80% or higher, preferably at least 85% or higher, more preferably at least 90% or higher, and particularly preferably at least 95% or higher.

It should be noted that, in the present specification, sequence identities of base sequences and amino acid sequences refer to those calculated using BLASTN, BLASTP, BLASTX, or TBLASTN (e.g., available from http://www.ncbi.nlm.nih.gov) with standard settings.

As used herein, a fragment of a polynucleotide refers to a polynucleotide having consecutive part of a base sequence of the biomarker, in which the length of the fragment is sufficient for specifically hybridizing with a probe for acquiring information regarding expression level of a biomarker as described later.

As used herein, a fragment of a protein refers to a polypeptide having a consecutive part of an amino acid sequence of the biomarker, in which the length of the fragment is sufficient for being specifically recognizable by an antibody or an aptamer (e.g., nucleic acid aptamer) for acquiring information regarding expression level of a biomarker as described later.

As used herein, information regarding expression level of a biomarker is not particularly limited as long as it is data that is acquired from a biological specimen using a method known in the art and that indicates expression level of each biomarker.

When the first and second biomarkers are polynucleotide markers, the information regarding expression levels of these biomarkers include, for example, a quantitative value of nucleic acid (DNA, mRNA, cDNA, or cRNA) acquirable using a method known in the art including nucleic acid amplification methods such as PCR methods, RT-PCR methods, real time PCR methods, and LAMP (Loop-mediated isothermal amplification) methods, and hybridization methods such as Southern hybridization and Northern hybridization, and microarray methods.

When the first and second biomarkers are polypeptide markers, the information regarding expression levels of these biomarkers include, for example, a quantitative value of protein acquirable using a method known in the art including immunoprecipitation techniques, Western blotting, ELISA method, and flow cytometry method using an antibody or a nucleic acid aptamer for acquiring information regarding expression level of a later described biomarker.

In embodiments of the present invention, a molecule that specifically hybridize with the first and second biomarkers as a polynucleotide marker can be used as a probe (hereinafter, the probe is also referred to as "expression level acquisition probe") for acquiring the information regarding expression level of the first and second biomarkers. The expression level acquisition probe may be one of a peptide probe and a nucleic acid probe capable of specifically hybridizing with the first and second biomarkers, which are a polynucleotide marker. As such probe, a nucleic acid probe is preferable, and a DNA probe is particularly preferable.

As used herein, "capable of specifically hybridizing" refers to an ability of the probe to hybridize with the first and second biomarkers as a polynucleotide marker under a stringent condition.

As used herein, stringent condition refers to a condition in which the level of hybridization of the probe with a target polynucleotide is detectably larger than that with polynucleotides besides the target polynucleotide (e.g., at least twice or higher than background).

It should be noted that, a stringent condition is ordinarily sequence dependent, and differs in various environments. Generally, a stringent condition is selected to have a temperature lower than a thermal melting point (Tm) of a specific sequence at prescribed ionic strength and pH, by approximately 5°C. This Tm is a temperature causing hybridization of, in an equilibratory manner, 50% of a probe to its target nucleic acid molecule having a base sequence complementary to that of the probe (under prescribed ionic strength pH, and nucleic acid composition).

Such condition may be a condition used for hybridization between polynucleotides in methods known in the art (e.g., a condition for hybridization between polynucleotides, as used in PCR methods, microarray methods, Southern blotting methods and the like.) Specifically, the condition is indicated as follows: pH of 7.0 to 9.0; a salt concentration in terms of Na ion of approximately 1.5 M or lower (more specifically, Na ion concentration (or other salts) of approximately 0.01 to 1.0 M) and a temperature of at least approximately 30°C. For example, a stringent condition in a microarray method includes hybridization at 37°C in 50% formamide, 1M NaCl, and 1% SDS, and rinsing at 60 to 65°C in 0.1 × SSC. Furthermore, a stringent condition in a PCR method includes a condition with pH of 7.0 to 9.0, 0.01 to 0.1 M Tris HCl, 0.05 to 0.15 M K ion concentration (or other salts), and at least approximately 55°C.

A person skilled in the art can appropriately determine a base sequence of the expression level acquisition probe so as to specifically hybridize with the marker, based on technical common knowledge in the art and the base sequences of the first and second biomarkers. Such sequences can be determined using, for example, generally available primer designing software (e.g., Primer3 (available from http://frodo.wi.mit.edu/cgi-bin/primer3/primer3.cgi), and DNASIS Pro (Hitachi Software Engineering Co., Ltd.)).

An expression level acquisition probe can be produced using a polynucleotide synthesizing method known in the art. The length of an expression level acquisition probe is ordinarily 5 to 50 nucleotides, and is preferably 10 to 40 nucleotides.

A single type or a combination of multiple types of the expression level acquisition probe may be used. For example, a microarray for acquiring the information regarding expression levels of the first and second biomarkers can be produced by fixing one or more types of the probe on a substrate using a method known in the art.

The expression level acquisition probe may be, for example, two or more types of primer sets for amplifying the first and second biomarkers using a nucleic acid amplification method.

In embodiments of the present invention, molecules capable of specifically binding to the first and second biomarkers as a polypeptide marker can be used for acquiring the information regarding expression levels of the biomarkers. Although such molecules may be either one of an aptamer and an antibody capable of specifically binding to the first and second biomarkers, such molecules are preferably antibodies (hereinafter, such antibody is also referred to as "expression level acquisition antibody").

The expression level acquisition antibody can be produced by, for example, a procedure known in the art as described next. Based on an amino acid sequence of a protein or a base sequence of a gene which is the first and second biomarkers, a DNA molecule encoding a protein having an amino acid sequence of one of the biomarkers is incorporated in a proper expression vector. The obtained expression vector is introduced in a proper host cell, and the obtained transformed cell is cultured to obtain the target protein. The obtained protein is purified to be used as an immunogen, and an appropriate mammal such as rat, mice, etc., is immunized using the immunogen and, if desired, together with an adjuvant. From spleen cells of the immunized animal, an antibody-producing cell that produces an antibody against the target immunogen is selected through screening. The obtained antibody-producing cell is fused with myeloma cell to obtain hybridomas, and through screening thereof, an antibody-producing hybridoma for producing an antibody that can specifically bind to a protein encoded by the gene can be obtained. By culturing the obtained antibody-producing hybridoma, an expression level acquisition antibody can be obtained.

An aptamer (hereinafter, the aptamer is also referred to as "expression level acquisition aptamer") that can be used for acquiring the information regarding expression levels of the first and second biomarkers can be produced by, for example, a procedure known in the art as described next. An aptamer capable of specifically binding to the target protein (the first and second biomarkers as polypeptides) can be selected by creating a nucleic acid library having random nucleic acid base sequences using a method known in the art, and applying thereto Systematic Evolution of Ligands by Exponential Enrichment method (SELEX method) or the like.

The expression level acquisition probe, antibody, and aptamer may be labeled with a labeling substance ordinarily used in the art. By using the labeled probe, antibody, and aptamer, the information regarding expression levels of the first and second biomarkers can be acquired easily. Such labeling substance may be labeling substances ordinarily used in the art, including radioisotopes such as ³²P, ³⁵S, ³H, and ¹²⁵I, fluorescent substances such as fluorescein, Alexa Fluor (Registered trademark), enzymes such as alkaline phosphatase and horseradish peroxidase, biotin, avidin, and streptavidin.

In embodiments of the present invention, when acquiring the information regarding expression levels of the first and second biomarkers using a nucleic acid amplification method, examples of the information include optically measured values (fluorescence intensity, turbidity, absorbance, etc.) of a reaction solution after amplification. The information regarding expression levels of the first and second biomarkers also include, in a nucleic acid amplification method, the number of cycles (or time period) required for an optically measured value to reach a predetermined reference value, the number of cycles (or time period) required for an amount of change of an optically measured value to reach a predetermined reference value, and a quantitative value of mRNA acquired from an optically measured value (or number of cycles) and a standard curve.

It should be noted that a predetermined reference value can be set as appropriate in accordance with the type of the data. For example, when the data is the number of cycles, an amount of change of the fluorescence intensity indicating a logarithmic growth of nucleic acid amplification reaction may be set as the predetermined reference value.

When acquiring the information regarding expression levels of the first and second biomarkers using a microarray method, examples of the information include signal intensity (fluorescence intensity, coloring intensity, change in amount of current, etc.) derived from specific hybridization between, for example, a nucleic acid probe on a microarray, and mRNA of one of the genes or nucleic acid (cDNA, cRNA, etc.) derived from the mRNA. The information regarding expression levels of the first and second biomarkers also include a quantitative value of mRNA acquired from the signal intensity and a standard curve.

In a preferable embodiment of the present invention, the information regarding expression levels of the first and second biomarkers are numerical values indicating a correlation with the morbidity status of RA and obtained by mathematically combining quantitative values of mRNA of the first and second biomarkers. Examples of the numerical value indicating correlation with the morbidity status of RA include a value acquired by applying multivariate analysis on the quantitative values of mRNA of the first and second biomarkers. It should be noted that such multivariate analysis is known in the art, and examples thereof include multiple logistic regression analysis etc. In addition, multivariate analysis can be executed by a computer using commercially available software such as STAT Flex ver.6 (Artech Co., Ltd.).

Therefore, a preferable embodiment of the present invention further includes a step of acquiring a value through multivariate analysis conducted on the acquired information (e.g., quantitative value of mRNA) regarding expression levels of the first and second biomarkers. In this case, presence of rheumatoid arthritis in a subject is evaluated based on the acquire value. Examples of the value acquired through multivariate analysis include a predictive value that can be obtained through multiple logistic regression analysis.

In the evaluation method of the present invention, presence of RA in a subject is evaluated based on the information acquired from a biological specimen regarding expression levels of the first and second biomarkers.

As used herein, "evaluating presence of rheumatoid arthritis in a subject" refers to determining whether or not a subject is affected with RA, and when the subject is affected with RA, refers to quantifying the degree (disease activity) of the symptom.

In addition, when the subject is receiving treatment against RA, the scope of the present invention also includes determining whether there has been improvement or advancement in the disease activity, i.e., monitoring therapeutic effect on RA in the subject.

Therefore, another embodiment of the present invention is a method for monitoring rheumatoid arthritis, the method comprising the steps of: acquiring, from a biological specimen obtained from a subject who has received treatment against RA, information regarding expression level of a first biomarker which is SLC16A4, and information regarding expression level of at least one second biomarker which is SLCO2B1; and monitoring a therapeutic effect on rheumatoid arthritis in the subject based on the acquired information regarding expression levels of the first and second biomarkers.

The embodiment described above may further include a step of acquiring a value through multivariate analysis such as multiple logistic regression analysis from the acquired information (e.g., quantitative value of mRNA) regarding expression levels of the first and second biomarkers. In this case, therapeutic effect on rheumatoid arthritis in the subject is monitored based the acquired value.

As described above, in RA patients, the present inventors have found high levels of expression of each of the genes of SLC16A4 which is the first biomarker, and SLCO2B1, PTPRM, SHB, and ATP9A as second biomarkers. Therefore, presence of RA in a subject may be evaluated by determining whether or not the information regarding expression levels of the first and second biomarkers indicate high expression levels.

For example, in a case where the information regarding expression levels of the first and second biomarkers are quantitative values of mRNA or protein, when the quantitative values are large, it is possible to determine that the subject is affected with RA or monitor that aggravation of disease activity of RA has occurred. On the other hand, when the quantitative values are small, it is possible to determine that the subject is not affected with RA or monitor that alleviation of disease activity of RA has occurred. Furthermore, based on the quantitative values, it is also possible to quantify the degree of symptom by acquiring a numerical index (e.g., a numerical value indicating correlation with morbidity status of RA, acquired through multivariate analysis such as logistic regression analysis) indicating disease activity of RA in the subject.

As described above, evaluation of presence of RA by a measured value itself can be conducted empirically through accumulation of data. However, when a subject is determined negative by a test with anti-CCP antibody but is suspected to be affected with RA, evaluation with higher accuracy is sometimes demanded. In such a case, the determination of whether or not the information regarding expression levels of the first and second biomarkers indicate high expression levels for both of them is preferably made from a result of comparing the information regarding expression levels and cutoff values depending on the type of the information.

For example, in a case where the information regarding expression levels of the first and second biomarkers are quantitative values of mRNA, it is possible to determine that the acquired information regarding expression levels of the first and second biomarkers indicate high expression levels for both of them, when the quantitative values for both the first and second biomarkers are equal to or larger than predetermined cutoff values. In this case, with the evaluation method of the present invention, it is possible to determine that the subject is affected with RA or monitor that aggravation of disease activity of RA has occurred.

On the other hand, when the quantitative values of both the first and second biomarkers are smaller than the predetermined cutoff values, it is possible to determine that the acquired information regarding expression levels of the first and second biomarkers do not indicate high expression levels for both of them. In this case, with the evaluation method of the present invention, it is possible to determine that the subject is not affected with RA or monitor that alleviation of disease activity of RA has occurred.

The "predetermined cutoff values" used in the evaluation method of the present invention can be configured as appropriate depending on the type of data regarding the expression levels. That is, the cutoff values may be empirically configured as values indicating specific expression levels that enable clear distinction between RA patients and healthy individuals. For example, when the information regarding expression levels of the first and second biomarkers are quantitative values of mRNA, respective quantitative values of mRNA are acquired from biological specimens obtained from RA patients and biological specimens obtained from healthy individuals, and mRNA levels that enable clear distinction between RA patients and healthy individuals can be configured as the predetermined cutoff values.

In a preferable embodiment of the present invention, when a predictive value (p) acquired through multiple logistic regression analysis conducted on the information regarding expression levels of the first and second biomarkers (e.g., quantitative values of mRNA) is equal to or larger than a predetermined cutoff value, it is possible to determine that the subject is affected with RA or monitor that aggravation of disease activity of RA has occurred. On the other hand, when the predictive value (p) is smaller than the predetermined cutoff value, it is possible to determine that the subject is not affected with RA or monitor that alleviation of disease activity of RA has occurred.

It should be noted that, the predetermined cutoff value in this embodiment can be calculated using, for example, commercially available software such as STAT Flex ver.6 (Artech Co., Ltd.) and can be determined using Youden index.

### [2. Biomarker Set for Evaluating Presence of Rheumatoid Arthritis]

A biomarker set for evaluating presence of rheumatoid arthritis (hereinafter, also simply referred to as "biomarker set") of the present disclosure includes a first biomarker that is a polynucleotide having a base sequence of a gene designated as SLC16A4, a mutated form thereof, or a fragment of those, and a second biomarker that is a polynucleotide having a base sequence of a gene designated as at least one selected from the group consisting of SLCO2B1, PTPRM, SHB, and ATP9A, a mutated form thereof, or a fragment of those.

Furthermore, the biomarker set of the present disclosure may be a set of polypeptide markers. Therefore, in another aspect of the present disclosure, a biomarker set includes a first biomarker that is a protein encoded by a gene designated as SLC16A4, a functionally equivalent mutated form thereof, or a fragment of those, and a second biomarker that is a protein encoded by a gene designated as at least one selected from the group consisting of SLCO2B1, PTPRM, SHB, and ATP9A, a functionally equivalent mutated form thereof, or a fragment of those.

The biomarker set of the present disclosure is suitably used for the evaluation method of the present invention, and can distinguish between RA patients and healthy individuals with high accuracy.

### EXAMPLES

Although the present invention will be described in detail by means of Examples, the present invention is not limited to these Examples.

### [Example 1]

### Identification of Biomarker Set for Evaluating Presence of RA

### 1. Isolation of CD4 Positive Cells from Peripheral Blood of Healthy Individuals and Rheumatoid Arthritis Patients

Peripheral blood was collected from healthy adults (n=20) and RA patients (n=33) in blood collection tubes NP-HE0557 (Nipro Corp.). The characteristics of the RA patient group include the following: 24 samples from females, 9 samples from males, ages ranging from 30 to 80 (average age of 56.9), and DAS (Disease Activity Score) 28 ranging from 1.8 to 5.2 (average of 3.2).

Magnetic beads bound with anti-CD4 antibody (hereinafter, referred to as "anti-CD4 antibody beads": Miltenyi Biotec Co., Ltd.) were added to the peripheral blood, and CD4 positive cells were isolated. It should be noted that this operation was conducted in accordance with the description in the attached document of the anti-CD4 antibody beads. The obtained CD4 positive cells were cryopreserved at -80°C until the next step of total RNA extraction.

### 2. Total RNA Extraction

By using RNeasy Plus Mini kit and RNeasy micro kit (Qiagen N. V.), total RNAs were extracted from the CD4 positive cells obtained at step 1 described above. It should be noted that the specific operations for those were conducted in accordance with the description in the attached documents of each of the kits.

### 3. Microarray Expression Analysis

By using Two-Cycle Target Labeling and Control Reagents (Affymetrix Inc.), total RNAs (10 to 100 ng) of respective cells extracted in step 2 described above were reverse transcribed into cDNAs, and the cDNAs were transcribed into biotinylated cRNAs. Next, the biotinylated cRNAs (20µg) were amplified and then fragmented. It should be noted that the specific operation was conducted in accordance with the description in the attached instruction of the kit.

The biotinylated cRNAs (15µg) derived from the respective cells obtained above were used as a sample and were each added to a GeneChip Human Genome U-133 Plus 2.0 Array (Affymetrix Inc.), and microarrays were transferred to a GeneChip Hybridization Oven 640 (Affymetrix Inc.) to allow hybridization for 16 hours under a condition of 45°C and 60 rpm.

After hybridization, the microarrays were rinsed and fluorescently labeled using a GeneChip Fluidic Station 450 (Affymetrix Inc.), and the microarrays were scanned using a GeneChip Scanner 3000 7G (Affymetrix Inc.) to acquire fluorescence intensity data.

### 4. Selection of Th17 Specific Genes that Vary Significantly between Healthy Individual Group and RA Patient Group

Fluorescence intensities indicating mRNA expression levels of each gene obtained at step 3 above were corrected by dividing those with a fluorescence intensity indicating a mRNA expression level of GAPDH gene which is a housekeeping gene, to obtain information (hereinafter, referred to as "expression level") regarding expression levels of each of the genes. Then, regarding these expression levels, a comparison was conducted between a healthy individual group and an RA patient group regarding genes (142 genes) that had been found as Th17 cell detection markers by the present inventors to be specifically expressed in Th17 cells. It should be noted that the genes that are specifically expressed in Th17 cells had been confirmed (International Application WO2011/013753) by the present inventors to be expressed at a level approximately three times of the expression levels of other Th cells (Th1, Th2, and Treg cells).

Of the 33 samples from the RA patient group, 20 samples were selected through random sampling using a computer program, and genes whose expression levels were significantly increased in the selected 20 samples than in the 20 samples from the healthy individual group were extracted. Here, a gene whose expression level has increased significantly is a gene indicating "p value < 0.05" in a Mann-Whitney test conducted for the healthy individual group and the RA patient group, and whose value of "(average expression level in RA patient group) / (average expression level in healthy individual group)" is 2 or higher. It should be noted that the random sampling and Mann-Whitney test were conducted using STAT Flex ver.6 (Artech Co., Ltd.).

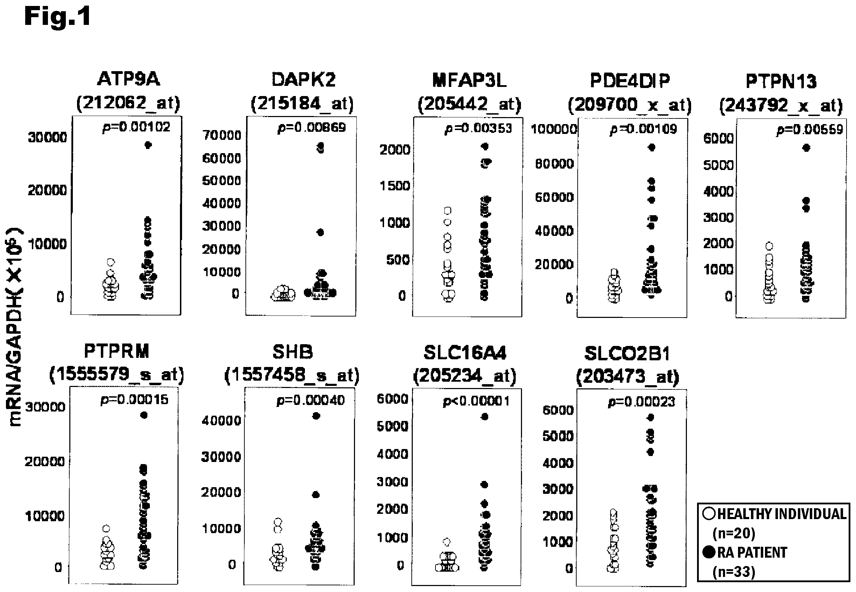

Random sampling from the RA patient group and extraction of genes whose expression levels have increased significantly were each repeated 6 times, and 9 genes whose expression levels were significantly increased in all of the 6 trials were identified as Th17 cell specific genes that vary significantly between the healthy individual group and the RA patient group. Expression levels of these 9 genes in the healthy individual group and the RA patient group were shown in FIG. 1. In FIG. 1, an open circle (○) represents a healthy individual, and a solid circle (●) represents an RA patient. Furthermore, the number described within a parenthesis below the name of each of the biomarkers is an ID number of a probe-set mounted on the Hunman Genome U-133 Plus 2.0 Array (Affymetrix Inc.).

In FIG. 1, although the above described 9 genes tend to show high expression in the RA patient group, there are many samples from RA patients that cannot be distinguished from those of healthy individuals only by a single gene itself. Therefore, it can be understood that it is difficult to use any of the 9 genes by itself as a biomarker capable of distinguishing between healthy individuals and RA patients with high accuracy.

### 5. Identification of Biomarker Set for Evaluating Presence of RA

For the purpose of finding from the 9 genes described above a combination of biomarkers capable of distinguishing between healthy individuals and RA patients with high accuracy, a biomarker set was selected by the following procedure.

From a sample group (n=53) obtained by combining the healthy individual group (n=20) and the RA patient group (n=33), 3 sample sets were created through random sampling without replacement, and number of samples (n) is 18 for one of these sample sets (n=18); 18 for another (n=18) and 17 for the other (n=17). Next, among the 3 sets of sample sets, 2 sets were used as training sets, and the remaining 1 set was used as a test set to create a training-test set. This was repeated for 3 times to ultimately create 3 sets of test-training sets having different combinations.

With respect to the 9 genes obtained at step 4 above, multiple logistic regression analysis was conducted for all conceivable gene combinations. The multiple logistic regression analysis was conducted on each of the 3 sets of training-test sets, and 5 regression models that can differentiate between healthy individuals and RA patients were extracted. Calculation formulae (regression formulae) for the predictive values (p) of the extracted regression models, and the combination of genes included in each of the models were shown in the following Table 2. In the formulae, [SLC16A4], [SLCO2B1], [PTPRM], [ATP9A], and [SHB], are values indicating expression levels of each of the genes obtained at step 4 described above. It should be noted that the sampling without replacement and the multiple logistic regression analysis were conducted using STAT Flex ver.6 (Artech Co., Ltd.), and each coefficient in the regression formulae was calculated using data of all samples (n=53) obtained by combining the healthy individual group and the RA patient group.

**[Table 2]**

| **Regression model** | **Gene combination** | **Calculation formula for predictive value (p)** |
|---|---|---|
| 1 | SLC16A4 PTPRM SHB | p = 1/(1+exp(-x)) |
| | | x = -5.9216 + 0.00530 × [SLC16A4] + 0.00041 × [PTPRM] + 0.00030 × [SHB] |
| 2 | SLC16A4 SLCO2B1 | p = 1/(1+exp(-x)) |
| | | x = -4.3021 + 0.00488 × [SLC16A4] + 0.00149 × [SLCO2B1] |
| 3 | SLC16A4 SLCO2B1 ATP9A | p = 1/(1+exp(-x)) |
| | | x = -5.6934 + 0.00494 × [SLC16A4] + 0.00146 × [SLCO2B1] + 0.00038 × [ATP9A] |
| 4 | SLC16A4 SLCO2B1 PTPRM | p = 1/(1+exp(-x)) |
| | | x = -6.5452 + 0.00536 × [SLC16A4] + 0.00151 × [SLCO2B1] + 0.00041 × [PTPRM] |
| 5 | SLC16A4 SLCO2B1 SHB | p = 1/(1+exp(-x)) |
| | | x = -5.0055 + 0.00502 × [SLC16A4] + 0.00138 × [SLCO2B1] + 0.00017 × [SHB] |

### 6. Comparison of Ability of Each Biomarker Set to Differentiate between Healthy Individuals and RA Patients

From the expression levels of each of the samples in the RA patient group (n=33) and in the healthy individual group (n=20), the predictive value (p) of each of the samples was calculated using the 5 regression models obtained in step 5 described above. It should be noted that the predictive values were calculated from the formulae set forth in Table 2.

Next, ROC curves (Receiver Operating Characteristic curves) were created based on the calculated predictive value of each of the samples to identify a cutoff value for each of the regression models using Youden index. A calculated predictive value was determined to be positive when it was equal to or larger than the cutoff value, and was determined to be negative when it was smaller than the cutoff value. Sensitivity and specificity were calculated based on this determination result.

It should be noted that creation of the ROC curve, calculation of the cutoff value, and calculation of sensitivity and specificity were conducted using STAT Flex ver.6 (Artech Co., Ltd.).

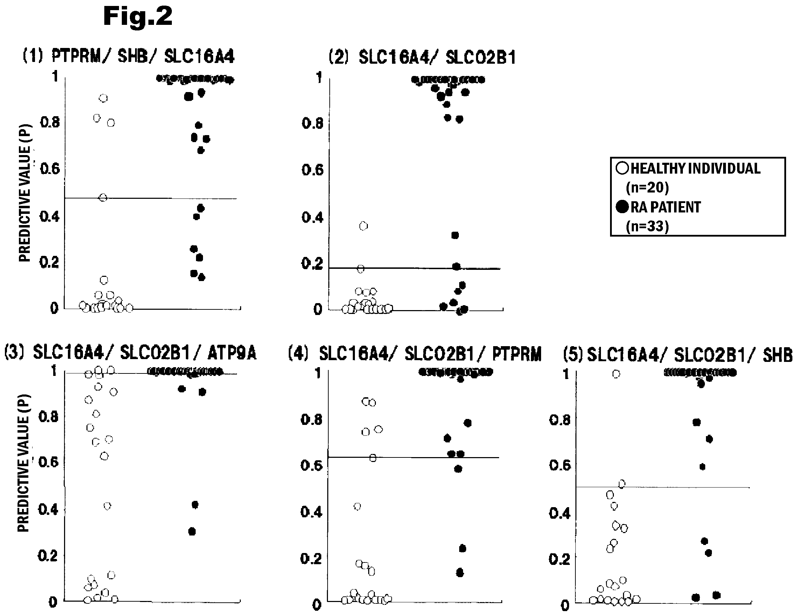

The sensitivity and specificity, and the cutoff value of each of the regression models were shown in Table 3. In addition, for each of the regression models, the predictive values of the samples from the healthy individual group and the RA patient group were each shown in FIG. 2. In FIG. 2, an open circle (○) represents a healthy individual, and a solid circle (●) represents an RA patient. Furthermore, a cutoff value was shown in each of the scatter diagrams in FIG. 2.

**[Table 3]**

| **Regression model** | **Combination of Biomarkers** | **Sensitivity (%)** | **Specificity (%)** | **Cutoff value** |
|---|---|---|---|---|
| (1) | SLC16A4, PTPRM, SHB | 81.8 | 85.0 | 0.47654 |
| (2) | SLC16A4, SLCO2B1 | 81.5 | 95.0 | 0.19767 |
| (3) | SLC16A4, SLCO2B1, ATP9A | 84.8 | 90.0 | 0.97840 |
| (4) | SLC16A4, SLCO2B1, PTPRM | 90.9 | 80.0 | 0.61524 |
| (5) | SLC16A4, SLCO2B1, SHB | 87.8 | 95.0 | 0.51101 |

From FIG. 2, it can be understood that the evaluation method of the present invention using the biomarker set can distinguish between healthy individuals and RA patients with high sensitivity and high specificity. In addition, from Table 3, it can be understood that the evaluation method of the present invention can distinguish and detect RA patients from healthy individuals with 80% or higher sensitivity and specificity by using any of the biomarker set.

In each of the scatter diagram in FIG. 2, there were some samples from RA patients whose predictive values were smaller than the cutoff values. Table 4 shows disease activity (DAS28) of RA patients whose samples (8 samples) had a predictive value that was smaller than a cutoff value in any of the 5 regression models.

**[Table 4]**

| Sample | DAS28 |
|---|---|
| RA55 | 2.8 (Low value) |
| RA56 | 2.1 (Remission) |
| RA57 | 2.7 (Low value) |
| RA60 | 4.0 (Low value) |
| RA61 | 3.1 (Low value) |
| RA65 | 2.4 (Remission) |
| RA352 | 2.4 (Remission) |
| RA355 | 3.1 (Low value) |

As can be seen in Table 4, among 8 samples that had a value smaller than a cutoff value, 7 samples had a DAS28 that was a low value or a value at remission level. From this, it can be understood that almost all RA patients who had a value smaller than a cutoff value had low RA disease activity. Since all of these RA patients had been receiving treatment, it was thought that disease activity of RA was reduced due to effective treatment, and, as a response, resulted in a low predictive value. As a result, it was suggested that the evaluation method of the present disclosure using the above described biomarker set can identify, as about the same as a healthy individual, a patient whose symptoms have been alleviated because of treatment against RA. Therefore, it was suggested that the evaluation method of the present disclosure can be applied for monitoring therapeutic effect and disease activity.

### [Example 2]

### Differentiation Analysis of Healthy Individuals and Anti-CCP Antibody Negative RA Patients

An examination was conducted whether or not RA patients who had tested negative with anti-CCP antibody but was affected with RA (hereinafter, referred to as "anti-CCP antibody negative RA patients") can be distinguished from healthy individuals using the evaluation method of the present disclosure. Using expression levels of each sample from the healthy individual group (n=20) and the anti-CCP antibody negative RA patient group (n=10) and regression model (2) (combination of biomarkers: SLC16A4, SLCO2B1) identified in Example 1, predictive values (p) of each of the samples were calculated. The predictive values were calculated using the formula set forth in Table 2 of Example 1.

Next, a ROC curve was created, and a cutoff value of each of the regression models was identified using Youden index. A calculated predictive value was determined positive when it was equal to or larger than the cutoff value, and was determined negative when it was smaller than the cutoff value. Sensitivity and specificity were calculated based on this determination result. It should be noted that creation of the ROC curve, calculation of the cutoff value, and calculation of sensitivity and specificity were conducted using STAT Flex ver.6 (Artech Co., Ltd.).

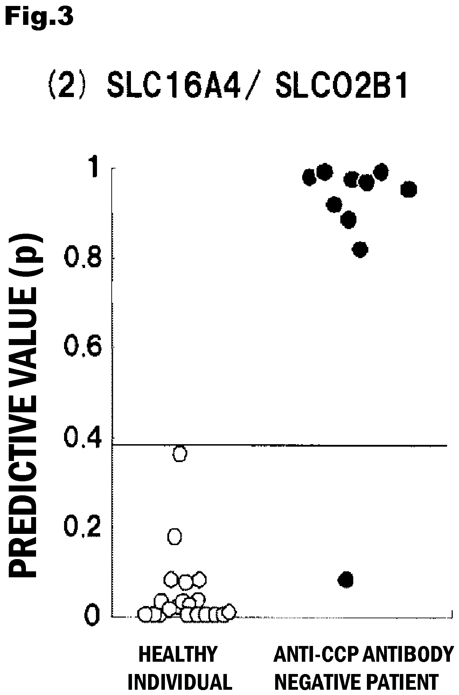

The sensitivity, specificity, and cutoff value obtained in the present embodiment were shown in Table 5. In addition, predictive values of the healthy individual group and the anti-CCP antibody negative RA patient group obtained in the present embodiment were shown in FIG. 3. In FIG. 3, an open circle (○) represents a healthy individual, and a solid circle (●) represents an RA patient. Furthermore, a cutoff value is shown in the scatter diagram in FIG. 3.

**[Table 5]**

| | Sensitivity (%) | Specificity (%) | Cutoff value |
|---|---|---|---|
| Regression model (2) | 90.0 | 100.0 | 0.37999 |

From FIG. 3, it can be understood that the evaluation method of the present disclosure can distinguish RA patients who had been determined negative with a conventional anti-CCP antibody test from healthy individuals with high sensitivity and high specificity. With this, it can be understood that the evaluation method of the present disclosure can diagnose RA more accurately when being used together with conventional RA diagnosis and testing methods.

## Claims

1. A method for evaluating presence of rheumatoid arthritis, comprising the steps of:
acquiring, from a biological specimen obtained from a subject, information regarding expression level of a first biomarker which is SLC16A4, and information regarding expression level of at least one second biomarker which is SLCO2B1; and
evaluating presence of rheumatoid arthritis in the subject based on the information regarding expression levels of the acquired first and second biomarkers.

2. The method according to claim 1, wherein the first biomarker is a polynucleotide having a base sequence of a gene designated as SLC16A4, a mutated form thereof, or a fragment of those.

3. The method according to claim 1, wherein the second biomarker at least includes a polynucleotide having a base sequence of a gene designated as SLCO2B1, a mutated form thereof, or a fragment of those.

4. The method according to claim 1, wherein the first biomarker is a protein encoded by a gene designated as SLC16A4, a functionally equivalent mutated form thereof, or a fragment of those.

5. The method according to claim 1, wherein the second biomarker at least includes a protein encoded by a gene designated as SLCO2B1, a functionally equivalent mutated form thereof, or a fragment of those.

6. The method according to any one of claims 1 to 5, wherein the biological specimen is a specimen containing a cell.

7. The method according to claim 6, wherein the cell is a cell in blood.

8. A method for monitoring rheumatoid arthritis, the method comprising the steps of:
acquiring, from a biological specimen obtained from a subject who has received treatment against rheumatoid arthritis, information regarding expression level of a first biomarker which is SLC16A4, and information regarding expression level of at least one second biomarker which is SLCO2B1; and
monitoring a therapeutic effect on rheumatoid arthritis in the subject based on the acquired information regarding expression levels of the first and second biomarkers.

## Patentansprüche

1. Verfahren zum Bewerten des Vorliegens von rheumatoider Arthritis, umfassend die folgenden Schritte:
Erwerben von Informationen bezüglich des Expressionsniveaus eines ersten Biomarkers, bei dem es sich um SLC16A4 handelt, und von Informationen bezüglich des Expressionsniveaus von mindestens einem zweiten Biomarker, bei dem es sich um SLCO2B1 handelt, von einer von einem Individuum erhaltenen biologischen Probe; und
Bewerten des Vorliegens von rheumatoider Arthritis in dem Individuum auf Grundlage der Informationen bezüglich der Expressionsniveaus des erworbenen ersten und des zweiten Biomarkers.

2. Verfahren nach Anspruch 1, wobei es sich bei dem ersten Biomarker um ein Polynukleotid mit einer Basensequenz eines Gens mit der Bezeichnung SLC16A4, einer mutierten Form davon oder eines Fragments von diesen handelt.

3. Verfahren nach Anspruch 1, wobei der zweite Biomarker mindestens ein Polynukleotid mit einer Basensequenz eines Gens mit der Bezeichnung SLCO2B1, einer mutierten Form davon oder eines Fragments von diesen beinhaltet.

4. Verfahren nach Anspruch 1, wobei es sich bei dem ersten Biomarker um ein Protein handelt, das von einem Gen mit der Bezeichnung SLC16A4, einer funktionell äquivalenten mutierten Form davon oder einem Fragment von diesen codiert wird.

5. Verfahren nach Anspruch 1, wobei der zweite Biomarker mindestens ein Protein beinhaltet, das von einem Gen mit der Bezeichnung SLCO2B1, einer funktionell äquivalenten mutierten Form davon oder einem Fragment von diesen codiert wird.

6. Verfahren nach einem der Ansprüche 1 bis 5, wobei es sich bei der biologischen Probe um eine Probe, die eine Zelle enthält, handelt.

7. Verfahren nach Anspruch 6, wobei es sich bei der Zelle um eine im Blut befindliche Zelle handelt.

8. Verfahren zum Verfolgen von rheumatoider Arthritis, wobei das Verfahren die folgenden Schritte umfasst:
Erwerben von Informationen bezüglich des Expressionsniveaus eines ersten Biomarkers, bei dem es sich um SLC16A4 handelt, und von Informationen bezüglich des Expressionsniveaus von mindestens einem zweiten Biomarker, bei dem es sich um SLCO2B1 handelt, von einer von einem Individuum erhaltenen biologischen Probe; und
Verfolgen einer therapeutischen Wirkung auf rheumatoide Arthritis in dem Individuum auf der Grundlage der erworbenen Informationen in Bezug auf die Expressionsniveaus des ersten und des zweiten Biomarkers.

## Revendications

1. Méthode d'évaluation de la présence de la polyarthrite rhumatoïde, comprenant les étapes suivantes :
acquisition, à partir d'un échantillon biologique obtenu auprès d'un sujet, d'informations concernant le niveau d'expression d'un premier biomarqueur qui est SLC16A4, et d'informations concernant le niveau d'expression d'au moins un deuxième biomarqueur qui est SLCO2B1 ; et
évaluation de la présence de la polyarthrite rhumatoïde chez le sujet en se basant sur les informations relatives au niveau d'expression des premier et deuxième biomarqueurs acquis.

2. Méthode selon la revendication 1, où le premier biomarqueur est un polynucléotide présentant une séquence de bases d'un gène désigné comme SLC16A4, de l'une de ses formes mutées ou d'un fragment de celles-ci.

3. Méthode selon la revendication 1, où le deuxième biomarqueur inclut au moins un polynucléotide présentant une séquence de bases d'un gène désigné comme SLCO2B1, de l'une de ses formes mutées ou d'un fragment de celles-ci.

4. Méthode selon la revendication 1, où le premier biomarqueur est une protéine codée par un gène désigné comme SLC16A4, l'une de ses formes mutées fonctionnellement équivalentes ou un fragment de celles-ci.

5. Méthode selon la revendication 1, où le deuxième biomarqueur inclut au moins une protéine codée par un gène désigné comme SLCO2B1, l'une de ses formes mutées fonctionnellement équivalentes ou un fragment de celles-ci.

6. Méthode selon l'une quelconque des revendications 1 à 5, où l'échantillon biologique est un échantillon contenant une cellule.

7. Méthode selon la revendication 6, où la cellule est une cellule du sang.

8. Méthode de suivi de la polyarthrite rhumatoïde, la méthode comprenant les étapes suivantes :
acquisition, à partir d'un échantillon biologique obtenu auprès d'un sujet ayant reçu un traitement contre la polyarthrite rhumatoïde, d'informations concernant le niveau d'expression d'un premier biomarqueur qui est SLC16A4, et d'informations concernant le niveau d'expression d'au moins un deuxième biomarqueur qui est SLCO2B1 ; et
suivi d'un effet thérapeutique sur la polyarthrite rhumatoïde chez le sujet en se basant sur les informations acquises relatives aux niveaux d'expression des premier et deuxième biomarqueurs.
